# EUROPÄISCHE PATENTSCHRIFT

(11) **EP 0 875 567 B1**
(45) Veröffentlichungstag und Bekanntmachung des Hinweises auf die Patenterteilung: **19.11.2003**
(21) Anmeldenummer: 98106426.4
(22) Anmeldetag: 08.04.1998
(51) Int. Cl.: C12N 15/12, C07K 14/47, C12N 15/63, C12N 1/21, G01N 33/68, C07K 16/18, A61K 48/00

(54) **Myc-bindende Zinkfinger-Proteine, ihre Herstellung und ihre Verwendung**
Mycbinding Zinc-finger proteins, preparation and use thereof
Protéines à doigts de zinc se liant à l'oncogène mycm ainsi comment leur préparation et utilisation

(30) Priorität: 30.04.1997 DE 19718249
(43) Veröffentlichungstag der Anmeldung: 04.11.1998
(73) Patentinhaber: Prolifix Limited, Abingdon, Oxfordshire OX14 4RY (GB)
(72) Erfinder: Peukert, Karen, 35094 Lahntal-Sterzhausen (DE); Haenel, Frank, Dr., 07745 Jena (DE); Eilers, Martin, Prof. Dr., 35043 Marburg-Cappel (DE)
(74) Vertreter: Brasnett, Adrian Hugh

(56) Entgegenhaltungen:
- THOMAS C. SCHULZ ET AL.: "An unusual arrangement of 13 zinc fingers in the vertebrate gene Z13" BIOCHEMICAL JOURNAL, Bd. 311, Nr. 1, 1.Oktober 1995, Seiten 219-224, XP002117525
- N. TOMMERUP ET AL.: "Isolation and fine mapping of 16 novel human zinc finger-encoding cDNAs identify putative candidate genes for developmental and malignant disorders" GENOMICS, Bd. 27, Nr. 2, 20.Mai 1995, Seiten 259-264, XP002117526 -& R59u022 Datenbank Hs20647 Zugriffsnummer U20647; 6 März 1995 TOMMRUP N. ET AL.:"H uman zinc finger protein (ZNF151) mRNA, pa rtial cds." XP002117530
- ANGELIKA PHILIPP ET AL.: "Repression of Cyclin D1: a novel function of MYC" MOLECULAR AND CELLULAR BIOLOGY, Bd. 14, Nr. 6, Juni 1994, Seiten 4032-4043, XP002117527
- PEUKERT K ET AL: "An alternative pathway for gene regulation by Myc." EMBO JOURNAL, (1997 SEP 15) 16 (18) 5672-86., XP002117528
- SCHNEIDER A ET AL: "Association of Myc with the zinc-finger protein Miz -1 defines a novel pathway for gene regulation by Myc." CURRENT TOPICS IN MICROBIOLOGY AND IMMUNOLOGY, (1997) 224 137-46., XP002117529

## Beschreibung

Die vorliegende Erfindung betrifft ein Verfahren zur identifizierung von spezifischen Transkriptionsmodulierenden Substanzen mittels Myc bindenden Zinkfinger-proteine.

Myc ist ein spezifisch an DNA bindendes Protein. Es wird zur Familie der Helix-Loop-Helix/Leucin-Zipper (HLH/LZ) Transkriptionsfaktoren gezählt (Landschulz et al, 1988, Murre et al., 1989). Myc ist ein zentraler Transkriptionsaktivator, der mit dem Protein Max (Amati et al., 1993) einen Komplex bildet und durch diesen molekularen Mechanismus andere Gene aktiviert, beispielsweise alpha-Prothymosingen, Ornithindecarboxylasegen und cdc25A.

Von Schulz et al, 1995, wurde ein 13 Zinkfinger enthaltendes Protein aus der Maus beschrieben, dessen zelluläre Funktion jedoch unklar ist.

Aufgrund seiner Schlüsselstellung in der Transkription bietet Myc einen Ansatzpunkt zum Verständnis von zellulären, insbesondere von pathophysiologischen Prozessen.

Es bestand daher die Aufgabe, weitere Informationen über die molekulare Wirkungsweise von Myc, insbesondere über die Myc vermittelte Genrepression bereitzustellen.

Das Protein das die in SEQ ID No:2 dargestellten Aminosäuresequenz hat, besitzt dreizehn Zinkfingerdomänen.

Es weist folgende biologischen Eigenschaften auf:
. Spezifische Bindung an Myc,
. Transaktivierung des Adenovirus Major Late (AdML) Promoters,
. Transaktivierung des Cyclin D1 Promoters,
. durch Assoziation mit Myc wird die Transaktivierung gehemmt,
in Abwesenheit von Myc ist das Protein im wesentlichen im Cytosol assoziiert mit Mikrotubuli zu finden.

Proteine können aus der SEQ ID NO:2 dargestellten Struktur duch Substitution, Insertion oder Deletion von einem oder mehreren Aminosäuren abgeleitet werden, welche Proteine noch die wesentlichen biologischen Eigenschaften des durch SEQ ID NO:2 beschriebenen Proteins besitzen. Diese Proteine werden im folgenden Muteine genannt. Unter wesentlichen Eigenschaften wird die spezifische Bindung der Muteine an Myc verstanden.

Die oben aufgeführten Eigenschaften des durch SEQ ID NO:2 beschriebenen Proteins müssen nicht alle bei den Muteinen vorhanden sein, solange die spezifische Bindung an Myc gegeben ist. Bevorzugt sind jedoch diejenigen Muteine, die alle der oben aufgeführten Eigenschaften besitzen.

Die oben geschilderten Proteine eignen sich als Testsysteme zur Auffindung von potentiellen selektiven Transkriptionsmodulierenden Substanzen. Dies läßt sich besonderes gut testen, indem man die Fähigkeit der Proteine, mit Myc einen Proteinkomplex zu bilden, ausnützt. Ein weiterer Gegenstand der Erfindung ist daher ein Verfahren zur Identifizierung von spezifischen trankriptionsmodulierenden Substanzen, das folgende Schritte umfaßt:
(a) Inkubation eines ersten, die in SEQ ID NO:2 dargestellten aminosäuresequenz umfassenden Proteins, oder eines daraus durch Substitution, Insertion oder Deletion von einem oder mehreren amionsäureresten abgeleiteten, noch eine spezifische Bindung an Myc aufweisenden Muteins, dem Gen-produkt von myc unter Bedingungen, unter denen sich ein Proteinkomplex zwischen diesen beiden Proteinen ausbildet,
(b) Inkubation der beiden Proteine unter ansonst gleichen Bedingungen wie (a) jedoch in Anwesenheit einer oder mehrerer Substanzen, die auf spezifische transkriptionsmodulierende Aktivitäten zu testen sind,
(c) Ermitteln des Unterschiedes in der Proteinkoplexbildung zwischen (b) und (a),
(d) Auswahl solcher Substanzen, bei denen gemäß Schritt (b) eine andere Proteinkomplexbildung erhalten wurde als bei Schritt (a).

Ein weiterer Gegenstand der Erfindung sind verfrahren wie oben beschriebenen, wobei das besagte erste Protein ein aus einem Protein mit der in SEQ ID NO:2 dargestellten Aminosäuresequenz, durch Substitution, Insertion oder Deletion von einem oder mehreren Aminosäureresten abgeleitetes und die folgenden biologischen Eigenschaften aufweisendes Mutein ist: spezifische Bindung an Myc, Transaktivierung des Adenovirus Major Late Promoters, Transaktivierung des Cyclin D1 Promoters, und Hemmung der Transaktivierung durch Assoziation mit Myc.

Es lassen sich damit Substanzen auffinden, die die Protein komplexbildung zwischen den neuen Zinkfingerprotein und Myc fördern, aber auch solche, die sie unterbinden.

Die Anzahl der durch Insertion Substitution oder Deletion gegenüber dem durch SEQ ID NO:2 beschriebenen Protein veränderten Aminosäuren kann zwischen 1 und 100, bevorzugt zwischen 1 und 50 Aminosäuren variieren. Die Veränderungen können in einem kleineren Bereich des Moleküls konzentriert oder auch über das ganze Molekül verteilt sein.

Bevorzugte Veränderungen sind konservative Substitutionen, bei denen eine Aminosäure durch eine andere Aminosäure mit ähnlicher Raumerfüllung, Ladung oder Hydrophilie ersetzt wird.

Beispiele für solche konservativen Substitutionen sind
Ersatz von Arg durch Lys oder umgekehrt,
Ersatz von Arg durch His oder umgekehrt,
Ersatz von Asp durch Glu oder umgekehrt,
Ersatz von Asn durch Gln oder umgekehrt,
Ersatz von Cys durch Met oder umgekehrt,
Ersatz von Gly durch Ala oder umgekehrt,
Ersatz von Val durch Leu oder umgekehrt,
Ersatz von Val durch Ile oder umgekehrt,
Ersatz von Leu durch Ile oder umgekehrt,
Ersatz von Phe durch Tyr oder umgekehrt,
Ersatz von Phe durch Trp oder umgekehrt,
Ersatz von Ser durch Thr oder umgekehrt.

Die Veränderungen können auch kombiniert werden, z.B. eine oder mehrere Substitutionen mit Deletionen und/oder Insertionen.

Nukleinsäuresequenzen, die für die oben beschriebenen Proteine zu codieren benutzt können, sind bevorzugt DNA, insbesondere cDNA Sequenzen, in einzelsträngiger oder doppelsträngiger Form.

Bevorzugte Nukleinsäuresequenzen sind solche mit der in SEQ ID NO:1 dargestellten Sequenz und solche, die mit dieser Sequenz einen hohen Verwandschaftsgrad aufweisen, beispielsweise solche, die für das gleiche Protein codieren wie SEQ ID NO:1. Weitere bevorzugte Nukleinsäuresequenzen sind solche, die für ein Protein codieren, das 95% oder mehr Identität mit dem Protein der Sequenz SEQ ID NO:2 aufweist.

Die Herstellung der Proteine, die hierin beschrieben werden, erfolgt bevorzugt mit Hilfe gentechnischer Verfahren. Ein Wirtsorganismus, der die Erbinformation für die erfindungsgemäßen Proteine trägt, wird unter Bedingungen kultiviert, die die Expression des Proteins erlauben. Diese Bedingungen - wie Temperatur, Nährmedium, Zelldichte - hängen weitgehend von der Wahl des Wirtsorganismus ab. Solche Bedingungen sind jedoch dem Fachmann für die einzelnen Wirtsorganismen geläufig. Geiegnete Wirstorganismen sind Mikroorganismen, pflanziche oder tierische Zellen oder Lebewesen. Bevorzugte Wirtsorganismen sind eukaryontische Zellen und Lebewsen. Der Begriff Wirtsorganismus umfaßt auch beispeilsweise transgene Tiere und Pflanzen.

Die exprimierten Proteine werden anschließend, ggf. nach Aufbrechen des Wirtsorganismus, vom Wirtsorganismus abgetrennt und in reiner Form duch bekannte Methoden der Proteinreinigung, wie Fällung, Chromatographie, Elektrophorese in reiner Form isoliert.

Die weitere Ausgestaltung der Erfindung ist in den folgenden Beispeilen aufgeführt.

### Beispiel 1

Isolierung der DNA mit der durch SEQ ID NO:1 beschriebenen Struktur Vorausgegangene Arbeiten hatten gezeigt, daß die Integrität der Helix-Loop-Helix Domäne von Myc kritisch für die Genrepression durch Myc in stabilen Zellinien war (Philipp et al., 1994). Um neue Proteine zu identifizieren, die mit dem C-Terminus von Myc interagieren, wurde ein DNA-Fragment, das für die basische Region und die HLH/LZ Domäne (Aminosäuren 355-439 des humanen Myc) codiert, im Leserahmen an die DNA bindende Domäne von GAL4 ( Aminosäure 1-147) fusioniert und als Köder in einem "Two-Hybrid-Screen" (Fields and Song, 1989) benutzt.

2x10⁵ unabhängige Transformanden einer HeLa cDNA Bibliothek, markiert mit der GAL4 Aktivierungsdomäne, wurden gescreent. Ein Clon mit β-Galaktosidaseaktivität wurde weiter charakterisiert. Es wurde keine Interaktion zwischen dem von diesem Clon codierten Protein und der DNA Bindungsdomäne von GAL4 allein oder einer GAL4-BCY-1 Chimäre, die als Negativkontrolle benutzt wurde, festgestellt.

Die Interaktion mit Myc wurde aufgehoben durch Deletion der HLH-Domäne in Myc (370-412), nicht aber durch Insertion der vier Aminosäuren zwischen der HLH Domäne und dem Leucin-Zipper (In 412) oder durch Deletion des gesamten Leucin-Zippers (412-434). Eine spezifische Interaktion wurde auch nachgewiesen mit N-Myc aber keine mit MAX oder USF, zwei HLH-Proteinen, die mit Myc nahe verwandt sind.

cDNA-Moleküle mit voller Länge wurden durch ein 5'-RACE-Protokoll isoliert und sequenziert (SEQ ID NO:1). Sie codieren ein Protein mit 803 Aminosäuren (SEQ ID NO:2) mit einem theoretischen Molekulargewicht von 87,970 Dalton. Das Protein wurde Miz-1 für Myc-Interacting-Zincfinger-Protein-1 genannt.

Die Sequenzierung ergab, daß der isolierte Clon für ein Zinkfingerprotein mit 13 Zinkfingern codierte, 12 davon unmittelbar geclustert in der C-terminalen Hälfte des Proteins.

### Beispiel 2

### Herstellung von Muteinen

Ausgehend von der in SEQ ID NO:1 dargestellten Nukleinsäuresequenz können mit dem Fachmann geläufigen Methoden der Gentechnik Nukleinsäuren hergestellt werden, die für veränderte Proteine (Muteine) codieren. Die Herstellung der Muteine selbst erfolgt zweckmäßigerweise durch Expression einer Nukleinsäure in einem geeigneten Wirtsorganismus.

### Beispiel 3

### Assoziation des Proteins SEQ ID NO:2 mit Myc

Der C-Terminus des Proteins SEQ ID NO:2 (Aminosäure 269-803) wurde mit der Glutathion-Transferase (GST) (Smith and Johnson, 1988) fusioniert, das GST-Miz-1 Fusionsprotein gereinigt und mit in vitro synthetisiertem, radioaktiv markiertem Myc Protein inkubiert. Myc assoziiert spezifisch mit GST-Miz-1, jedoch nicht mit GST. Eine Mutante von Myc, der die HLH Domäne fehlt, konnte nicht mit GST-Miz-1 assoziieren. Radioaktiv markiertes Max interagiert weder mit GST-Miz-1 noch mit GST. Jedoch kann mit Hilfe von Myc Max an GST-Miz-1-Kügelchen in vitro binden, was dafür spricht, daß Miz-1 und Max mit unterschiedlichen Flächen der HLH-Domäne von Myc interagieren.

### Literaturverzeichnis

Amati, B., Brooks, M. W., Levy, N., Littlewood, T. D., Evan, G. I., and Land, H. (1993). Oncogenic activity of the c-Myc protein requires dimerization with Max. Cell 72, 233-245.
Fields, S., and Song, O. (1989). A novel genetic system to detect protein-protein interactions. Nature 340, 245-246.
Landschulz, W. H., Johnson, P. F., and McKnight, S. L. (1988). The leucine zipper: a hypothetical structure common to a new class of DNA binding proteins. Science 240, 1759-1764.
Murre, C., SchonleberMcCaw, P., and Baltimore, D. (1989). A new DNA binding and dimerization motif in immunoglobulin enhancer binding, daughterless, MyoD, and myc proteins. Cell 56, 777-783.
Philipp, A., Schneider, A., Väsrik, I., Finke, K., Xiong, Y., Beach, D., Alitalo, K., and Eilers, M. (1994). Repression of Cyclin D1: a Novel Function of MYC. Mol. Cell. Biol. 14, 4032-4043.
Schulz, T. C., Hopwood, B., Rathjen, P. D., and Wells, J. R. (1995). An unusual arrangement of 13 zinc fingers in the vertebrate gene Z13. Biochem. J. 311, 219-224.
Smith, D. B., and Johnson, K. S. (1988). Single-step purification of polypeptides expressed in Escherichia coli as fusions with glutathione-S-transferase. Gene 67, 31-40.

## Patentansprüche

1. Verfahren zur Identifizierung von spezifischen transkriptionsmodulierenden Substanzen, das folgende Schritte umfaßt:
(a) Inkubation eines ersten, die in SEQ ID NO:2 dargestellten Aminosäuresequenz umfassenden Proteins, oder eines daraus durch Substitution, Insertion oder Deletion von einem oder mehreren Aminosäureresten abgeleiteten, noch eine spezifische Bindung an Myc aufweisenden Muteins, mit dem Gen-produkt von Myc unter Bedingungen, unter denen sich ein Proteinkomplex zwischen diesen beiden Proteinen ausbildet,
b) Inkubation der beiden Proteine unter ansonst gleichen Bedingungen wie (a) jedoch in Anwesenheit einer oder mehrerer Substanzen, die auf spezifische transkriptionsmodulierende Aktivitäten zu testen sind,
c) Ermitteln des Unterschiedes in der Proteinkomplexbildung zwischen (b) und (a),
d) Auswahl solcher Substanzen, bei denen gemäß Schritt (b) eine andere Proteinkomplexbildung erhalten wurde als bei Schritt (a).

2. Verfahren gemäß Anspruch 1, wobei das besagte erste Protein ein aus einem Protein mit der in SEQ ID NO:2 dargestellten Aminosäuresequenz, durch Substitution, Insertion oder Deletion von einem oder mehreren Aminosäureresten abgeleitetes und die folgenden biologischen Eigenschaften aufweisendes Mutein ist: spezifische Bindung an Myc, Transaktivierung des Adenovirus Major Late Promotors, Transaktivierung des Cyclin Dl Promotors, und Hemmung der Transaktivierung durch Assoziation mit Myc.

3. Verfahren gemäß Anspruch 1, wobei das besagte erste Protein ein humanes Protein ist.

4. Verfahren gemäß Anspruch 1, wobei das besagte erste Protein mindestens 95 % Identität mit der in SEQ ID NO:2 dargestellten Sequenz besitzt.

## Claims

1. Method for identifying specific transcription-modulating substances, which comprises the following steps:
(a) incubating a first protein comprising the amino acid sequence depicted in SEQ ID NO: 2, or a mutein which is derived therefrom by substitution, insertion or deletion of one or more amino acid residues and still displays specific binding to Myc, with the Myc gene product under conditions under which a protein complex between these two proteins is formed,
(b) incubating the two proteins under conditions which are otherwise the same as in (a) but in the presence of one or more substances which are to be tested for specific transcription-modulating activities,
(c) determining the difference in the protein complex formation between (b) and (a),
(d) selecting those substances with which the protein complex formation in step (b) is different from that in step (a).

2. Method according to Claim 1, where the said first protein is a mutein which is derived from a protein having the amino acid sequence depicted in SEQ ID NO: 2 by substitution, insertion or deletion of one or more amino acid residues and displays the following biological properties: specific binding to Myc, transactivation of the adenovirus major late promotor, transactivation of the cyclin D1 promotor, and inhibition of transactivation by association with Myc.

3. Method according to Claim 1, where the said first protein is a human protein.

4. Method according to Claim 1, where the said first protein has at least 95% identity with the sequence depicted in SEQ ID NO: 2.

## Revendications

1. Procédé pour l'identification des substances spécifiques modulatrices de transcription, comprenant les étapes suivantes :
(a) l'incubation d'une première protéine comprenant la séquence d'acides aminés présentée dans la SEQ ID N°2 ou d'un mutant présentant encore une liaison spécifique à Myc, dérivé de celle-ci par substitution, insertion ou délétion d'un ou de plusieurs esters d'acides aminés, avec le produit génique de Myc dans des conditions où un complexe protéique se forme entre ces deux protéines,
(b) l'incubation des deux protéines dans des conditions sinon identiques au (a), cependant en présence d'une ou de plusieurs substances dont les activités spécifiques modulatrices de transcription doivent être testées,
c) la détermination de la différence dans la formation du complexe protéique entre (b) et (a),
d) la sélection des substances dans lesquels selon l'étape (b) une formation de complexe protéique différente de celle de l'étape (a) a été obtenue.

2. Procédé selon la revendication 1, dans lequel ladite première protéine est un mutant dérivé d'une protéine ayant la séquence d'acides aminés présentée dans la SEQ ID N°2, par substitution, insertion ou délétion d'un ou de plusieurs esters d'acides aminés et présentant les propriétés biologiques suivantes : liaison spécifique à Myc, transactivation du promoteur tardif majeur de l'adénovirus, transactivation du promoteur D1 de la cycline, et inhibition de la transactivation par association avec Myc.

3. Procédé selon la revendication 1, dans lequel ladite première protéine est une protéine humaine.

4. Procédé selon la revendication 1, dans lequel ladite première protéine possède une identité d'au moins 95 % avec la séquence présentée dans la SEQ ID N°2.


### SEQUENZPROTOKOLL

(1) ALGEMEINE INFORMATION:
   (i) ANMELDER:
      (A) NAME: BASF Aktiengesellschaft
      (B) STRASSE: Carl-Bosch-Strasse 38
      (C) ORT: Ludwigshafen
      (E) LAND: Bundesrepublik Deutschland
      (F) POSTLEITZAHL: D-67056
      (G) TELEPHON: 0621/6048526
      (H) TELEFAX: 0621/6043123
      (I) TELEX: 1762175170
   (ii) ANMELDETITEL: Myc-bindende Zinkfingerproteine
   (iii) ANZAHL DER SEQUENZEN: 2
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 2680 Basenpaare
      (B) ART: Nukleinsäure
      (C) STRANGFORM: Einzel
      (D) TOPOLOGIE: linear
   (ii) ART DES MOLEKÜLS: cDNS zu mRNS
   (iii) HYPOTHETISCH: NEIN
   (iii) ANTISENSE: NEIN
   (ix) MERKMALE:
      (A) NAME/SCHLÜSSEL: 5'UTR
      (B) LAGE: 1..159
   (ix) MERKMALE:
      (A) NAME/SCHLÜSSEL: CDS
      (B) LAGE: 160..2571
   (ix) MERKMALE:
      (A) NAME/SCHLÜSSEL: 3'UTR
      (B) LAGE: 2572..2680
   (xi) SEQUENZBESCHREIBUNG: SEQ ID NO: 1:
(2) INFORMATION ZU SEQ ID NO: 2:
   (i) SEQUENZ CHARAKTERISTIKA:
      (A) LÄNGE: 803 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Asp Phe Pro Gln His Ser Gln His Val Leu Glu Gln Leu Asn Gln
    1               5                  10                  15
   Gln Arg Gln Leu Gly Leu Leu Cys Asp Cys Thr Phe Val Val Asp Gly
               20                  25                  30
   Val His Phe Lys Ala His Lys Ala Val Leu Ala Ala Cys Ser Glu Tyr
           35                  40                  45
   Phe Lys Met Leu Phe Val Asp Gln Lys Asp Val Val His Leu Asp Ile
       50                  55                  60
   Ser Asn Ala Ala Gly Leu Gly Gln Met Leu Glu Phe Met Tyr Thr Ala
   65                  70                  75                  80
   Lys Leu Ser Leu Ser Pro Glu Asn Val Asp Asp Val Leu Ala Val Ala
                   85                  90                  95
   Thr Phe Leu Gln Met Gln Asp Ile Ile Thr Ala Cys His Ala Leu Lys
               100                 105                 110
   Ser Leu Ala Glu Pro Ala Thr Ser Pro Gly Gly Asn Ala Glu Ala Leu
           115                 120                 125
   Ala Thr Glu Gly Gly Asp Lys Arg Ala Lys Glu Glu Lys Val Ala Thr
       130                 135                 140
   Ser Thr Leu Ser Arg Leu Glu Gln Ala Gly Arg Ser Thr Pro Ile Gly
   145                 150                 155                 160
   Pro Ser Arg Asp Leu Lys Glu Glu Arg Gly Gly Gln Ala Gln Ser Ala
                   165                 170                 175
   Ala Ser Gly Ala Glu Gln Thr Glu Lys Ala Asp Ala Pro Arg Glu Pro
               180                 185                 190
   Pro Pro Val Glu Leu Lys Pro Asp Pro Thr Ser Gly Met Ala Ala Ala
           195                 200                 205
   Glu Ala Glu Ala Ala Leu Ser Glu Ser Ser Glu Gln Glu Met Glu Val
       210                 215                 220
   Glu Pro Ala Arg Lys Gly Glu Glu Glu Gln Lys Glu Gln Glu Glu Gln
   225                 230                 235                 240
   Glu Glu Glu Gly Ala Gly Pro Ala Glu Val Lys Glu Glu Gly Ser Gln
                   245                 250                 255
   Leu Glu Asn Gly Glu Ala Pro Glu Glu Asn Glu Asn Glu Glu Ser Ala
               260                 265                 270
   Gly Thr Asp Ser Gly Gln Glu Leu Gly Ser Glu Ala Arg Gly Leu Arg
           275                 280                 285
   Ser Gly Thr Tyr Gly Asp Arg Thr Glu Ser Lys Ala Tyr Gly Ser Val
       290                 295                 300
   Ile His Lys Cys Glu Asp Cys Gly Lys Glu Phe Thr His Thr Gly Asn
   305                 310                 315                 320
   Phe Lys Arg His Ile Arg Ile His Thr Gly Glu Lys Pro Phe Ser Cys
                   325                 330                 335
   Arg Glu Cys Ser Lys Ala Phe Ser Asp Pro Ala Ala Cys Lys Ala His
               340                 345                 350
   Glu Lys Thr His Ser Pro Leu Lys Pro Tyr Gly Cys Glu Glu Cys Gly
           355                 360                 365
   Lys Ser Tyr Arg Leu Ile Ser Leu Leu Asn Leu His Lys Lys Arg His
       370                 375                 380
   Ser Gly Glu Ala Arg Tyr Arg Cys Glu Asp Cys Gly Lys Leu Phe Thr
   385                 390                 395                 400
   Thr Ser Gly Asn Leu Lys Arg His Gln Leu Val His Ser Gly Glu Lys
                   405                 410                 415
   Pro Tyr Gln Cys Asp Tyr Cys Gly Arg Ser Phe Ser Asp Pro Thr Ser
               420                 425                 430
   Lys Met Arg His Leu Glu Thr His Asp Thr Asp Lys Glu His Lys Cys
           435                 440                 445
   Pro His Cys Asp Lys Lys Phe Asn Gln Val Gly Asn Leu Lys Ala His
       450                 455                 460
   Leu Lys Ile His Ile Ala Asp Gly Pro Leu Lys Cys Arg Glu Cys Gly
   465                 470                 475                 480
   Lys Gln Phe Thr Thr Ser Gly Asn Leu Lys Arg Gln Leu Arg Ile His
                   485                 490                 495
   Ser Gly Glu Lys Pro Tyr Val Cys Ile His Cys Gln Arg Gln Phe Ala
               500                 505                 510
   Asp Pro Gly Ala Leu Gln Arg His Val Arg Ile His Thr Gly Glu Lys
           515                 520                 525
   Pro Cys Gln Cys Val Met Cys Gly Lys Ala Phe Thr Gln Ala Ser Ser
       530                 535                 540
   Leu Ile Ala His Val Arg Gln His Thr Gly Glu Lys Pro Tyr Val Cys
   545                 550                 555                 560
   Glu Arg Cys Gly Lys Arg Phe Val Gln Ser Ser Gln Leu Ala Asn His
                   565                 570                 575
   Ile Arg His His Asp Asn Ile Arg Pro His Lys Cys Ser Val Cys Ser
               580                 585                 590
   Lys Ala Phe Val Asn Val Gly Asp Leu Ser Lys His Ile Ile Ile His
           595                 600                 605
   Thr Gly Glu Lys Pro Tyr Leu Cys Asp Lys Cys Gly Arg Gly Phe Asn
       610                 615                 620
   Arg Val Asp Asn Leu Arg Ser His Val Lys Thr Val His Gln Gly Lys
   625                 630                 635                 640
   Ala Gly Ile Lys Ile Leu Glu Pro Glu Glu Gly Ser Glu Val Ser Val
                   645                 650                 655
   Val Thr Val Asp Asp Met Val Thr Leu Ala Thr Glu Ala Leu Ala Ala
               660                 665                 670
   Thr Ala Val Thr Gln Leu Thr Val Val Pro Val Gly Ala Ala Val Thr
           675                 680                 685
   Ala Asp Glu Thr Glu Val Leu Lys Ala Glu Ile Ser Lys Ala Val Lys
       690                 695                 700
   Gln Val Gln Glu Glu Asp Pro Asn Thr His Ile Leu Tyr Ala Cys Asp
   705                 710                 715                 720
   Ser Cys Gly Asp Lys Phe Leu Asp Ala Asn Ser Leu Ala Gln His Val
                   725                 730                 735
   Arg Ile His Thr Ala Gln Ala Leu Val Met Phe Gln Thr Asp Ala Asp
               740                 745                 750
   Phe Tyr Gln Gln Tyr Gly Pro Gly Gly Thr Trp Pro Ala Gly Gln Val
           755                 760                 765
   Leu Gln Ala Gly Glu Leu Val Phe Arg Pro Arg Asp Gly Ala Glu Gly
       770                 775                 780
   Gln Pro Ala Leu Ala Glu Thr Ser Pro Thr Pro Pro Glu Cys Pro Pro
   785                 790                 795                 800
   Pro Ala Glu